# EUROPEAN PATENT APPLICATION

(11) **EP 3 699 299 A1**
(43) Date of publication of application: **26.08.2020**
(21) Application number: 19305215.6
(22) Date of filing: 22.02.2019
(51) Int. Cl.: C12Q 1/6886

(54) **PROCESS FOR CLASSIFICATION OF GLIOMA**

(71) Applicant: Hospices Civils de Lyon, 69002 Lyon (FR); Université Claude Bernard Lyon 1, 69100 Villeurbanne (FR)
(72) Inventor: PONCET, Delphine, 69150 Decines (FR)
(74) Representative: Regimbeau

(57) **Abstract**

The invention relates to an *in vitro* process for classifying a glioma, comprising the following steps:
a. Measuring, from a glioma sample, the following two parameters:
i. The Alternative Lengthening of Telomeres (ALT) status ; and
ii. The telomere length status;

b. Optionally, determining the isocitrate dehydrogenase genes mutation status (IDH status) of said glioma;
c. Optionnally, determining the histological grade of said glioma;
d. Based on the data obtained in steps (a) (b) and (c), classifying said glioma in one of the five following classes: oligodendroglioma -like, glioblastoma IDHwt -like, glioblastoma IDHmt -like, low-grade astrocytoma -like, and other gliomas.

## Description

### FIELD OF THE INVENTION

The present invention concerns the classification of tumor brains and the choice of therapeutic options useful for treating patients with tumor brains, based on said classification.

### BACKGROUND OF THE INVENTION

A glioma is a type of tumor deriving from the glial cells of the brain or the spine. Gliomas represent about 30 % of all brain and central nervous system tumors, and 80 % of the malignant brain tumors.

Malignant gliomas are graded from grade II to grade IV, although benign gliomas are designated as gliomas of grade I.

According to the 2016 WHO classification (4^{th} Edition) of tumors of the central nervous system (CNS), summarized in (Louis *et al*., 2016), malignant tumors of the CNS are classified according to immuno-histological and molecular criteria into different categories:
- diffuse astrocytic and oligodendroglial tumors;
- others astrocytic tumors;
- ependymal tumors;
- choroid plexus tumors;
- neuronal and mixed neuro-glial tumors;
- tumors of the pineal region;
- embryonal tumors (including medulloblastoma, CNS neuroblastoma);
- tumors of the cranial and paraspinal nerves; and
- "other" gliomas.

Gliomas are defined as tumors of the category "diffuse astrocytic and oligodendroglial tumors". In this 4^{th} edition of the WHO classification, gliomas are defined with molecular biomarkers, and then subclassed into the following subclasses: the oligodendroglioma (OD), the astrocytoma (A), the glioblastoma (GBM), the diffuse midline glioma and the oligoastrocytoma.

Molecular features define theses subclasses:
- Complete deletion of both the short arm of chromosome 1 (1p) and the long arm of chromosome 19 (19q), designated as 1p/19q co-deletion, is the molecular genetic signature of oligodendrogliomas;
- Histone mutations (*H3.3 or H3.1 K27M* mutation and G34R/V H3.3 mutation) have been identified in diffuse midline glioma; presence of a histone mutation was recognized to portend an adverse prognosis, regardless of the histological grade of the lesion;
- Isocitrate dehydrogenase 1 or 2 mutations are observed in low-grade astrocytoma (anaplasic astrocytoma (AA) or diffuse astrocytoma (AD)), oligodendroglioma and secondary GBM, also named IDH mutated GBM (IDHmt). Primary GBM do not show mutation of IDH, and are classified as IDHwt GBM.

When no molecular marker is available, glioma are classified as being "NOS" (for Not Otherwise Specified). In this circumstance, glioma is designated with the term "oligoastrocytoma".

Histological criteria such as vascularization, necrosis or proliferation further refine each subclass into different grades: diffuse (grade II) or anaplasic (grade III) glioma. Grade IV, the most advanced stage, defines glioblastoma.

Because of their diffusely infiltrating nature, grade II to IV gliomas cannot be completely resected and are not curable by surgical excision.

Each subclass of glioma is defined by a median time of overall survival (OS) of the patients affected by said glioma. A brief summary of features of each subclass is presented below.
1. Oligodendrogliomas are believed to originate from the oligodendrocytes of the brain or from a glial precursor cell. They occur primarily in adults (median age at onset is 45 years) but are also found in children. Oligodendrogliomas are slowly growing, and therefore patients have a prolonged survival compared to other glioma. They benefit from less aggressive therapeutic approach. Median survival times for patients affected with oligodendroglioma are of about 15 years for grade II, and 3.5 years for grade III.
2. Astrocytomas (grade II and III) can occur in most parts of the brain. They originate in a particular kind of glial cells, star-shaped brain cells called astrocytes. People can develop astrocytomas at different ages. For grade II to IV astrocytomas, despite decades of therapeutic research, curative treatment is still non-existent. The median overall survival is of about 5 to 8 years for grade II astrocytoma, and about 3 years for grade III astrocytoma.
3. Grade IV astrocytoma is designed as "multiform glioblastoma" (GBM). It is the most frequent adult brain tumor, and one of the most aggressive tumors among all human cancers. The extremely infiltrative nature of this tumor makes complete surgical removal impossible.

Among glioblastoma, clinicians distinguish primary glioblastoma that have an IDHwt status, and secondary glioblastoma that present a mutation in IDH 1 and/or IDH 2 (IDHmt) and that are supposed to derive from low-grade, IDHmt astrocytoma.

Patients affected with a secondary glioblastoma present a better median overall survival than those affected with primary glioblastoma: the overall survival of patients with primary glioblastoma ranges from 7 to 15 months, whereas the overall survival with secondary glioblastoma is about 2 years and a half.

Diffuse astrocytoma IDHmt (grade II), anaplasic astrocytoma IDHmt (grade III), glioblastoma IDHmt (grade IV), glioblastoma IDHwt (grade IV) are mainly reported in adults.
4. Diffuse midline glioma are grade IV tumors that tend to occur in children and young adult; they are characterized with the presence of a histone mutation. Diffuse midline glioma tends to spread out and invade neighboring tissue. These tumors are associated with an overall dismal prognosis, with a median overall survival of less than 12 months.
5. Oligoastrocytoma is a subclass of gliomas that present with an appearance of mixed glial cell origin, astrocytoma and oligodendroglioma. This class is considered to be obsolete by several scientifics, since it designates morphologically ambiguous tumors that should actually be classified into astrocytomas or oligodendrogliomas. With the identification of the molecular biomarker "co-deletion of 1p/19q", these gliomas can now be classified into one the following category:
   a. Oligodendroglioma if the co-deletion is present;
   b. Astrocytoma if no co-deletion is identified.

Histopathological classification is the basis of the World Health Organization (WHO) classification; however, it suffers from a high variability of interpretation from one practician to another. This is also due to the fact that tumors from the same category are highly heterogeneous. Consequently, therapeutic strategies may be wrongly chosen, if the glioma is incorrectly classified.

Another group of patients, not referenced in the current WHO's classification, consists in grade II and III glioma with astrocytoma features (based on morphological and immuno-histological parameters) but without any IDH mutation, this last feature being not expected according to the WHO's classification.

This "astrocytoma IDHwt" subclass is particularly heterogeneous in terms of response to treatment and overall survival, with a much more severe prognosis than the IDH mutated astrocytoma. No guideline is available for their treatment.

Beside histological features and specific molecular biormarkers, an additional defining feature for gliomas is the characterization of the telomere maintenance mechanisms (TMMs).

Telomeres are DNA-protein complexes present at the end of chromosomes in eukaryotic cells, which play a crucial role in cellular survival. Indeed, in healthy cells, a gradual shortening of telomere happens at each replicative cycle. When telomeres reach a critical size, cells stop to proliferate and enter in senescence. Most cancer cells maintain the length of their telomeres by the reactivation of telomerase, or through the telomerase-independent alternative lengthening of telomeres (ALT) mechanism.

The international application WO 2017/127803 discloses a glioma classification method based on multiple molecular features, comprising:
i) IDH mutation status,
ii) DNA methylation cluster,
iii) RNA cluster,
iv) Telomere length,
v) Telomere maintenance, and
vi) At least one biomarker, in particular selected among the following biomarkers:
   an amplification of Epidermal Growth Factor Receptor, a mutation in the protein p53, an IDH mutation, the co-deletion 1p19q, a chromosome 7 amplification coupled with a chromosome 10 deletion, a Cyclin-dependent kinase 4 (CDK4) amplification coupled with a Cyclin Dependent Kinase Inhibitor 2A (CDKN2A) deletion, a chromosome 19 (chrI9) amplification coupled with a chromosome 20 (chr20) amplification, a B-raf gene mutation coupled with a Neurofibromin 1 (NF1) mutation.

This method is based on the measure of at least six parameters, including molecular parameters. In view of (i) the quantity of biological material, (ii) the high cost and (iii) the time-consuming techniques that are necessary for measuring these parameters, this classification process would not be feasible in an usual clinical practice. Moreover, the timing for choosing a therapeutic strategy would be too long in regard to the duration of this diagnosis process.

A relevant classification of gliomas is the key for improving the therapeutic strategies and hopefully the clinical outcomes.

Currently, hospital services classify gliomas according to histological features and on the basis of available molecular biomarkers (such as mutation of TERT, mutation of histones, mutation of IDH, amplification of EGFR, deletion of CDKN2A). This classification process is fastidious, expensive, and variable from one hospital to another, since highly dependent on the pictures interpretation of the pathologist.

Since the current classification process is not satisfying, active research is currently conducted in order to identify and classify as precisely as possible gliomas, in a clinical environment, for helping health practitioners to choose the best therapeutic approach for patients.

Ideally, an efficient classification process would answer to the following criteria:
- It would be based on reproducible and reliable techniques;
- It would give homogeneous classification when performed by different services or hospitals;
- It could be realized with a few amount of DNA and, if necessary, with a poor quality sample of DNA;
- It would allow the classification of outsiders tumors, with no specific features, currently classified as "oligoastrocytoma" or "IDHwt astrocytoma"; and
- It would be easy, cheap and rapid, to give a quick answer to the clinician.

The present invention discloses a process of classification of gliomas that present all the advantages listed above.

### SUMMARY OF THE INVENTION

The present invention relates to an *in vitro* process for classifying a glioma, comprising the following steps:
a. Measuring, from a glioma sample, the following two parameters:
   i. The Alternative Lengthening of Telomeres (ALT) status ; and
   ii. The telomere length status;
b. Optionally, determining the isocitrate dehydrogenase genes mutation status (IDH status) of said glioma;
c. Optionally, determining the histological grade of said glioma;
d. Based on the data obtained in steps (a) (b) and (c), classifying said glioma in one of the five following classes: oligodendroglioma-like, glioblastoma IDHwt-like, glioblastoma IDHmt-like, low-grade astrocytoma-like and other gliomas.

Among said five classes, gliomas of low grades may be further refined as being of grade II or III, as a function of the histological grading determined in step (c).

In a specific implementation of this process, the parameters (a.i) and (a.ii) are measured, and then each parameter is compared to at least one threshold value.

In another specific implementation of this process, the ALT status in (a.i) is measured by performing a C-circle assay coupled to a telomere-specific PCR, thereby obtaining a C-circle value and selecting the ALT status of the glioma after comparison of said C-circle value to threshold values, determining four classes : "ALT -", "ALT intermediate", "ALT +", and "ALT++".

In another specific implementation of this process, the telomere length status in (a.ii) is measured by quantifying the telomeric DNA with a telomere-specific PCR, thereby obtaining a T-length value and selecting the telomere length status of the glioma after comparison of said T-length value with threshold values determining the 3 following classes: "short", "intermediate" and "long".

The present invention also concerns an *in vitro* process for reclassifying a glioma of the group "astrocytoma IDHwt", according to the WHO's classification, comprising the following steps:
a. Measuring, for said astrocytoma IDHwt, the following two parameters :
   i. The Alternative Lengthening of Telomeres (ALT) status ; and
   ii. The telomere length status;
b. Optionally, determining the histological grade of said glioma;
c. Based on the data obtained in steps (a) and (b), classifying said "astrocytoma IDHwt-like" in one of the following classes:
   oligodendroglioma-like, glioblastoma IDHwt-like, glioblastoma IDHmt-like,
   low-grade astrocytoma-like, and other gliomas.

Among said five classes, gliomas of low grades may be further refined as being of grade II or III as a function of the histological grading.

The present invention also concerns a process for choosing a therapeutic strategy for treating a glioma, comprising the steps of:
a) Implementing the *in vitro* process for classifying said glioma as described above; and
b) Based on said classification, choosing the more adapted therapeutic strategy for the patient affected by said glioma.

The present invention also concerns a process for adapting a therapeutic strategy for treating a glioma, comprising the steps of:
a) Implementing the *in vitro* process for classifying said glioma as described above; and
b) Based on said classification, adapting the therapeutic strategy for the patient affected by said glioma.

The present invention also concerns a computer program product comprising code instructions for implementing a process as defined above, for classifying a glioma.

The present invention also concerns a kit for the implementation of the processes as described above, comprising:
- Reagents suitable for performing a C-circle assay;
- Reagents suitable for performing a Telomere-specific PCR; and
- Adequate internal controls comprising genomic DNA from ALT+ cells and from ALT- cells.

The present invention also concerns an inhibitor of the telomere maintenance mechanism for its use in the treatment of a glioma, wherein said glioma has been previously classified according to the process as described above.

### BRIEF DESCRIPTION OF THE FIGURES

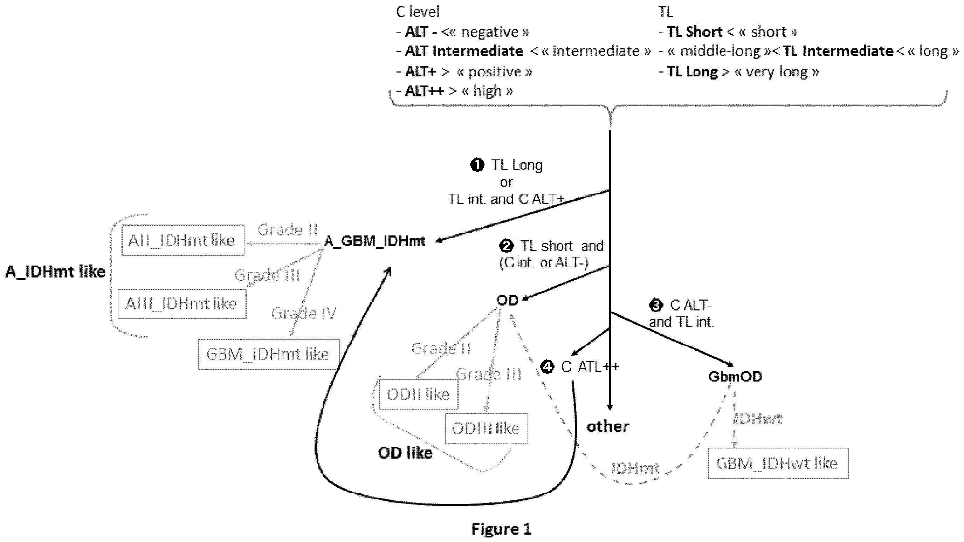

**Figure 1****. Schematic representation of the classification steps**
   The following abbreviations are used:
   TL: T-length value; long, int. and short designate the three possible TL status;
   C: C-circle value; int.: intermediate; ALT++, ALT+, ALT int. and ALT- designate the four possible ALT status;
   IDH: IDH status is chosen among IDHwt (no mutation) or IDHmt (at least one mutation in IDH1 and/or IDH2)
   Classification of gliomas "like" according to the algorithm of the invention: OD : oligodendroglioma; OD II: oligodendroglioma grade II; OD III: oligodendroglioma grade III; GbmOD: GBM or OD; GBM_IDHwt : primary glioblastoma; GBM_IDHmt: secondary glioblastoma; A_GBM_IDHmt: astrocytoma or secondary glioblastoma (mutated for IDH); A_IDHmt: astrocytomas; AIII_IDHmt : astrocytoma grade II; AIII_IDHmt : astrocytoma grade III.

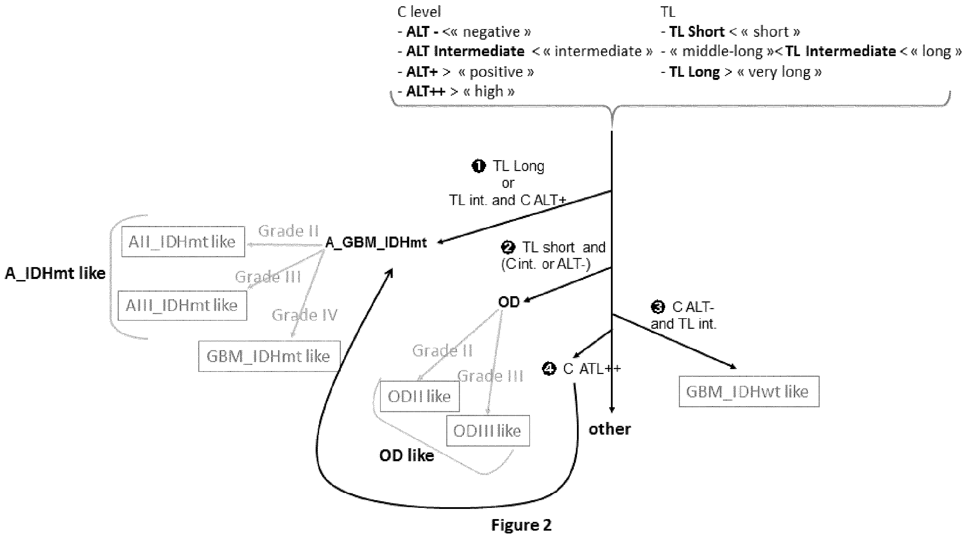

**Figure 2****. Schematic representation of the classification steps for an astrocytoma IDHwt**
   The same abbreviations than in figure 1 are used.
**Figure 3****. Standard curve for calculation of qPCR efficiency**
   qPCR were led using DNA from an ALT positive tumor (left : TeloPCR, right : qPCR targeting the reference housekeeping gene 36B4).
   (A) representative amplification curves are shown with (+) or without (-) pre-incubation with the enzyme φ29, as shown by the corresponding arrows. Telomere length is determined in the φ29-, and the c-circle value is calculated by using +φ29 and - φ29 values. Note that the same amount of genomic DNA is used in the two conditions (same curves for 36B4).
   (B) DNA from an ALT positive tumor was diluted and the CT value (y) is plotted against the log-transformed dilution factor (x) (TeloPCR on the left, and qPCR against 36B4 on the right). Note that the correlation coefficient (R²) is nearly 1 for both qPCR.

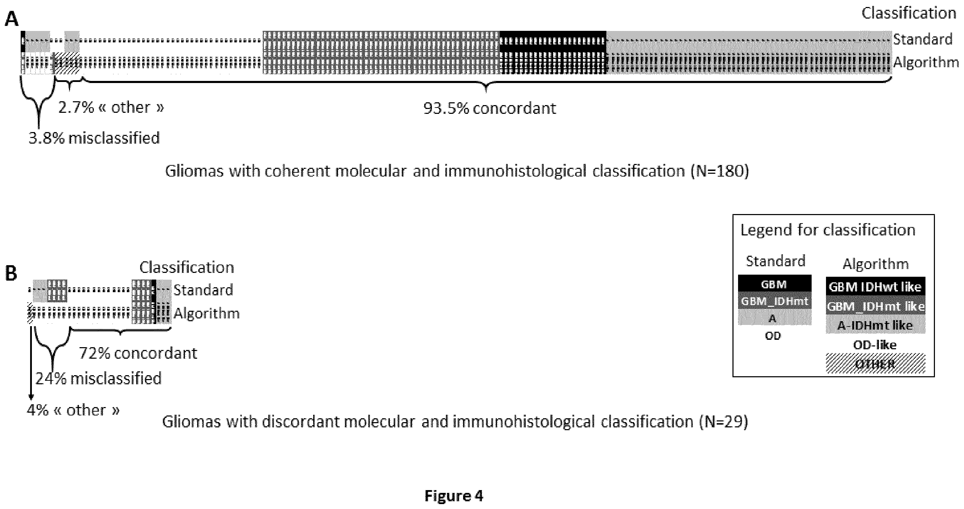

**Figure 4****. Comparison of the diagnosis value of the two classifications**
   Gliomas (with the exception of anaplastic astrocytoma IDHwt) have been classified according to the standard algorithm according to the WHO 2016 classification (upper line), or with the algorithm of the invention (bottom line).
   The algorithm of the invention allows a separation into five subclasses: "oligodendroglioma-like", "glioblastoma IDHwt-like", "glioblastoma IDHmt-like", "low-grade astrocytoma -like" and "other" (the code is depicted in the legend).
   (A) Only patients with molecular biomarkers in agreement with the immuno-histochemical classification are analyzed (N=180), the two classifications are concordant for 93.5% of gliomas, are different (noted misclassified) for 3.8%, and 2.7% of gliomas are classified as "others".
   (B) Considering only the gliomas with discordant molecular biomarkers and immuno-histochemical classification (N=29), the algorithm is in agreement with histological classification for 72% of gliomas.

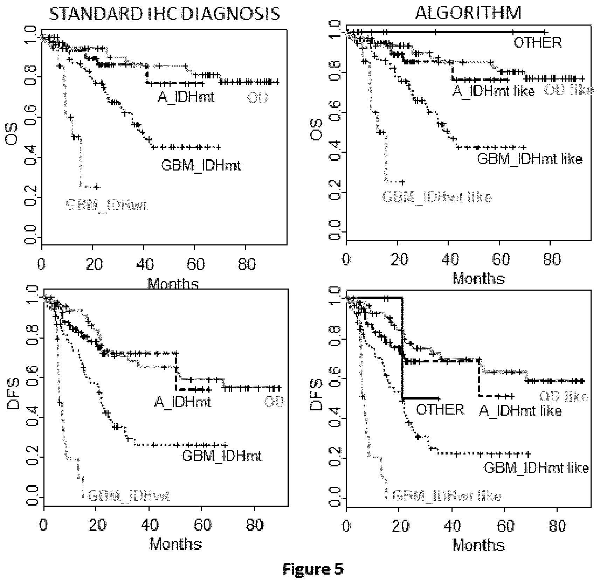

**Figure 5****. Comparison of the results curves of median overall survival and median disease-free survival obtained after standard classification and classification according to the process of the invention**
   Gliomas (with the exception of anaplastic astrocytoma IDHwt) have been classified according to the standard algorithm following the WHO 2016 classification (left curves), or with the algorithm of the invention (rigth curves).

The overall survival (OS, upper curves) and the disease free survival (DFS, bottom curves) have been followed for each patient. 210 patients were analyzed (Standard classification: 74 low-grade astrocytoma, 24 glioblastoma IDHwt, 57 glioblastoma IDHmt, 55 oligodendroglioma). Among these patients, 29 have discordant molecular parameters as regard with the immuno-histological classification.

The algorithm of the invention allows a separation into five subclasses: oligodendroglioma-like, glioblastoma IDHwt-like, glioblastoma IDHmt-like, low-grade astrocytoma -like (A_IDHmt like), and other gliomas.

The following abbreviations are used:
OD: oligodendroglioma; A_IDHmt: Astrocytoma_IDHmt; GBM_IDHmt: secondary glioblastoma (also called glioblastoma IDHmt); GBM_IDHwt: primary glioblastoma (also called glioblastoma IDHwt).

Note that the curves are highly similar regarding the overall survival, and that the algorithm is even more discriminant that the standard classification considering the low grade astrocytoma and the oligodendroglioma.

### DETAILED DESCRIPTION OF EMBODIMENTS OF THE INVENTION

"Glioma" is identified on the basis of histopathological analysis. Classification as a "glioma" is given by a health practician on the basis of:
- morphological criteria, such as cell shape and density, nuclear atypias, vascularisation, necrosis and mitosis number (determined on hematoxilin-eosin-labelled sections);
- immuno-histochemical labelling of different epitopes, such as GFAP, OLIG2, IDH1-R132H, ATRX, TP53, Ki67, and
- if available, molecular biomarker such as mutations of IDH1 and 2, and/or 1p/19q deletion (according to the WHO classification).

The present invention relates to an *in vitro* process for classifying a glioma, comprising the following steps:
a. Measuring, from a glioma sample, the following two parameters:
   i. The Alternative Lengthening of Telomeres (ALT) status ; and
   ii. The telomere length status;
b. Optionally, determining the isocitrate dehydrogenase genes mutation status (IDH status) of said glioma;
c. Optionally, determining the histological grade of said glioma;
d. Based on the data obtained in steps (a) (b) and (c), classifying said glioma in one of the five following classes: oligodendroglioma-like, glioblastoma IDHwt-like, glioblastoma IDHmt-like, low-grade astrocytoma like, and other gliomas.

This classification process allows the distinction of classes of gliomas, different from the 2016 WHO standard classification of tumors of the central nervous system.

The subclasses proposed by the present invention are the following:
∘ oligodendroglioma-like,
∘ glioblastoma IDHwt-like (also designated as primary glioblastoma),
∘ glioblastoma IDHmt-like (also designated as secondary glioblastoma),
∘ low-grade astrocytoma like, and
∘ other gliomas.

The phrase "low-grade astrocytoma" designates astrocytoma of grade II (diffuse astrocytoma) and of grade III (anaplastic astrocytoma).

The term "like" is used to mean that the patient in the group is expected to have similar response to treatment and overall survival as the group's name, but not necessarily have the corresponding immuno-histological or molecular parameters.

Each group of patients having glioma is defined by a median time of overall survival of said patients. For example, a patient whose glioma is classified in the class "glioblastoma IDHwt-like" can expect an overall survival time comprised between 7 and 15 months.

Advantageously, this classification process allows the re-classification of some gliomas that had been previously wrongly characterized, and consequently an incorrect expectation of median time of disease free survival and/or of overall survival had been announced to the patient. Furthermore, a non-adapted therapeutic strategy might be used for incorrectly classified patients.

In this case, the algorithm of the invention would allow to inform the patient that his/her median overall survival time would be 7 to 15 months (GBM IDHwt-like), instead of 15 years for an oligodendroglioma of grade II.

Also, the therapeutic strategies will not be the same, depending on the classification conclusions.

As presented in figure 4, the process of the invention allows a classification with a prognostic value at least equivalent to the standard (WHO's) classification. Advantageously, this classification process is easy to implement in a clinical environment, since it necessitates only DNA extracted from said glioma sample, for the measure of parameters (a.i), (a.ii) and optionally (b).

Optionally, the grading of the tumor is measured on the basis of immuno-histochemical analyses of the glioma sample.

It is understood that the process according to the invention comprises at least two steps (a) and (d) but might also comprise other supplementary steps, well known by the man skilled in the art, such as the optional steps (b) and (c), and also the detection of an EGFR amplification, the detection of a CDKN2A copy number loss, and/or the detection of chromosomal abnormalities like the gain in chromosome 7p correlated with loss of chromosome 10q (Inda et al., 2003).

In a specific embodiment of the invention, the process of the invention comprises both steps (a) and (d).

In a specific embodiment of the invention, the process of the invention comprises the three steps (a), (c) and (d).

In a specific embodiment of the invention, the process of the invention comprises the three steps (a), (b) and (d).

In a specific embodiment of the invention, the process of the invention comprises the four steps (a), (b), (c) and (d).

In a specific embodiment of the invention, the process of the invention consists in both steps (a) and (d).

In a specific embodiment of the invention, the process of the invention consists in three steps (a), (b) and (d).

In a specific embodiment of the invention, the process of the invention consists in three steps (a), (c) and (d).

In another specific embodiment of the invention, the process of the invention consists in the four steps (a) to (d).

### Origin of the DNA for the measure of parameters (a.i) (a.ii) and (b)

Surgery to remove as much of the tumor as possible is usually the first step in treating gliomas. After this procedure, a sample of the tumor is further analyzed.

If no surgery is performed, a stereotaxic biopsy may be realized to obtain a tumor sample. From this tumor sample, DNA is extracted by any technique well known by the man skilled in the art.

In an embodiment of the invention, the parameters (a.i), (a.ii) and (b) are measured on the basis of DNA extracted from a sample of said glioma, that has been recovered by any technique known by the man skilled in the art.

In most cases, glioma samples are not analyzed directly but are conserved before extraction of DNA. Typically such collected tissue samples are frozen or are processed as paraffin blocks. Advantageously, these tumor samples are annotated with clinical information on the patient.

In an embodiment of the invention, the parameters (a.i) (a.ii) and (b) of tumor samples are measured on the basis of DNA extracted from a glioma sample that has been conserved in paraffin or frozen.

Advantageously, the techniques for measuring parameters (a.i) (a.ii) and (b) of tumor samples can be realised on the basis of DNA extracted from a glioma sample that has been conserved in paraffin or frozen, even if said DNA is of poor quality and/or in a small amount.

### Implementation of the process on DNA extracted from blood of cerebrospinal fluid of a patient with glioma

In an aspect of the invention, the parameters (a.i) (a.ii) and (b) of tumor samples are measured on the basis of DNA of glioma cells, extracted from a blood sample or a cerebrospinal fluid sample from a patient having said glioma.

Glioma cells have a tendency to necrose and cell debris and/or free DNA circulate in blood and cerebrospinal fluid of patients affected by glioma. Moreover, cells designated as Circulating tumor cells (CTCs) issued from a primary tumor are able to circulate around the body in the blood circulation. From these cell debris, circulating DNA (circDNA) and/or CTCs, tumor-specific DNA can be purified and analyzed in order to assess the three parameters (a.i) (a.ii) and (b).

In this aspect of the invention, the term "glioma sample" designates the DNA from the glioma cells, that is obtained from the blood or from the cerebrospinal fluid of the patient.

This implementation of the process is advantageous since, in the preliminary steps not included in the process, the invasive step of biopsy for obtaining a tumor sample is avoided.

In this embodiment, the *in vitro* process for classifying a glioma comprises the following steps:
a. Measuring, from a glioma sample, i.e. from the DNA of glioma cells that has been obtained from the blood or from the cerebrospinal fluid of the patient, the following two parameters:
   i. The Alternative Lengthening of Telomeres (ALT) status ; and
   ii. The telomere length status;
b. Optionally, determining the isocitrate dehydrogenase genes mutation status (IDH status) of said glioma;
c. Based on the data obtained in steps (a) and (b), classifying said glioma in one of the five following classes: oligodendroglioma -like, glioblastoma IDHwt -like, glioblastoma IDHmt -like, low-grade astrocytoma -like, and other gliomas.

In this embodiment, no solid glioma sample is available. Therefore, the histological grading of the tumor cannot be determined. The process is therefore limited to the classification in five classes, without any further sub-classification of the gliomas according to their histological grade.

In a specific embodiment of the invention, the process of the invention comprises both steps (a) and (c).

In a specific embodiment of the invention, the process of the invention comprises the three steps (a), (b) and (c).

In a specific embodiment of the invention, the process of the invention consists in both steps (a) and (c).

In a specific embodiment of the invention, the process of the invention consists in three steps (a), (b) and (c).

### Threshold values

In a specific embodiment of the invention, the values of the parameters (a.i) and (a.ii), i.e. the ALT status and the telomere length status, are measured and then compared each one to at least one threshold value.

For the purposes of carrying out the above process, the term "threshold value" is intended to mean a value determined with a group of glioma samples that are well characterized, and whose ALT status and telomere length status have been previously determined.

Based on the results obtained for these groups of specific gliomas, inventors have defined threshold values for ALT status and telomere length that are defined below.

Other threshold values can be easily determined by those skilled in the art by means of their general knowledge.

Therefore, the value obtained in step (a.i) is compared to at least one ALT threshold value, in order to determine the ALT status of the glioma; and the value obtained in step (a.ii) is compared to at least one T-length threshold value, in order to determine the telomere length status of the glioma.

### Telomere maintenance mechanism (TMM)

In vertebrates, chromosome extremities, designed as telomeres, consist of:
- a repeated motif of sequence 5'-(TTAGGG)n-3', that is 10-15 kilobases long in human at birth, and a 3' G-rich single-stranded tail of 150 to 300 bases;
- six associated proteins which comprise the sheltering complex that promotes telomere protection, by ensuring stability and assisting specialized replication machinery for accurate extension of chromosome ends and recruitment of telomerase. The six proteins comprise three DNA-binding poteins (TRF1, TRF2, POT1) interconnected by three additional proteins (TIN2, ACD, RAP1).
Telomeres play vital roles in eukaryotic cells, in particular by limiting DNA replication and therefore avoiding unlimited cell proliferation, via their gradual shortening at each replicative cycle.

Telomerase is a ribonucleic reverse transcriptase enzyme, able to add the telomere repeat sequence to the 3' end of telomeres. It consists of a catalytic subunit called TERT (for Telomerase Reverse Transcriptase), an essential RNA component TERC that functions as the RNA template for the addition of the telomeric repeats, and a series of auxiliary components.

In most tumour cells, the TMM is linked to the reactivation of telomerase. Nevertheless, about 10% of tumour cells acquire immortality through the telomerase-independent alternative lengthening of telomeres (ALT) mechanism.

Glioma can therefore be classified as "ALT-dependent tumors", when the telomerase-independent mechanism is in place, and "ALT-independent tumors" when a telomerase-dependant mechanism is activated in the cell.

Within the ALT-dependent group, different subgroup can be identified: ALT intermediate, ALT+ and ALT ++.

The ALT-independent tumors define the ALT- subgroup, wherein no C circle is detected.

Any suitable assay for determining the ALT status of cells can be used for the implementation of the process.

Henson *et al.* (2009) have disclosed an assay based on partially single-stranded telomeric (CCCTAA)n DNA circles that allows a specific measurement of the ALT status. This assay is known as "the C-circle assay" or "CC assay".

Briefly, this C-circle assay can be summarized as follow:
Telomeric extrachromosomal DNA (ECT) is particularly enriched in ALT-dependant cells versus telomerase-positive or mortal cells. Among different species of ECT, C-circles is a specific and sensitive marker of the ALT process. A C-circle is composed of a circular DNA of C₃TA₂ sequence, partially double stranded with a short T₂AG₃ sequence. The technique of Rolling Circle Amplification (RCA) has been used to detect telomeric circles in ALT+ cells. The "CC-assay" involves the use of φ29, a highly processive DNA polymerase that is auto-primed by the partial G-strand (TTAGGG)n.

The amplified telomeric DNA is then quantified together with the genomic telomeric DNA after and before incubation with φ29. A ratio between these two quantities of DNA is calculated, and compared to the same ratio obtained in telomerase positive cells and in ALT-dependant cells. (Henson et al., 2017).

The amplified telomeric DNA can be quantified by all techniques well known by the man skilled in the art, such as hybridization and flow cytometry methods.

In a preferred embodiment of the invention, the quantification of the amplified telomeric DNA circles is performed as described in (Gil & Coetzer, 2004) with a real-time polymerase chain reaction (PCR) - based method, and particularly with a telomere-specific PCR.

A "C-circle" value, expressed in arbitrary units, equal to the following ratio:
Quantity of total telomeric DNA (including RCA-amplifiedC-circles) / Quantity of total telomeric DNA (without amplification of C-circle content)
is measured by the means described above for each glioma sample.

This value is then compared to at least three thresholds values, previously defined according to the general knowledge of the man skilled in the art.

In particular, the inventors have defined four thresholds as follow:
- A threshold said "high";
- A threshold said "intermediate"
- A threshold said "positive"
- A threshold said "negative".

In a specific embodiment of the invention, in the *in vitro* process, the ALT status in (a.i) is determined by performing a C-circle assay coupled to a telomere-specific PCR, thereby obtaining a C-circle value and selecting the ALT status of the glioma from:
- ALT++, when the C-circle value is superior to the threshold value "high";
- ALT+, when the C-circle value is superior to the threshold value "positive";
- ALT intermediate, when the C-circle value is inferior to the threshold value "intermediate", and
- ALT-, when the C-circle value is inferior to the threshold value "negative".

In a specific embodiment of the invention, the *in vitro* process for classifying a glioma comprises the following steps:
a. Measuring, from a glioma sample, the following two parameters:
   i. The Alternative Lengthening of Telomeres (ALT) status ; and
   ii. The telomere length status;
b. Optionally, determining the isocitrate dehydrogenase genes mutation status (IDH status) of said glioma;
c. Optionally, determining the histological grade of said glioma;
d. Based on the data obtained in steps (a) (b) and (c), classifying said glioma in one of the five following classes: oligodendroglioma -like, glioblastoma IDHwt -like, glioblastoma IDHmt -like, low-grade astrocytoma -like, and other gliomas,
wherein the ALT status in (a.i) is measured by performing a C-circle assay coupled to a telomere-specific PCR, thereby obtaining a C-circle value and selecting the ALT status of the glioma.

### Telomere length status

Measurement of telomere length is the second essential parameter of the process, measured in step (a.ii) of the process of the invention.

The original method for determining telomere length employed Southern hybridization to determine the mean terminal restriction fragment length. However this technique was labor-intensive, time-consuming and necessitates high amount of DNA (over 1µg), not available in standard clinical use. Various alternative techniques have since been proposed, including slot blots, next generation sequencing, hybridization protection assays and flow cytometry.

Although any technique for determining the telomere length status might be employed in the implementation of the process of the invention, in a preferred embodiment, telomere length status in (a.ii) is determined by quantifying the telomeric DNA with a telomere-specific PCR.

In particular, the real-time quantitative PCR described in (Gil & Coetzer, 2004) can be used for this quantification.

According to this embodiment, the quantification technique comprises the following steps:
- Quantitative PCR reactions are initiated with addition of primers specifics for telomeres (TeloPCR) and for the used housekeeping gene(s) such as 36B4 (36B4 PCR) to samples of total DNA;
- The efficiency of TeloPCR (E_{Telo}) and 36B4 (E_{36B4}) PCR is determined on the basis of standard curves generated from two reference DNA samples, one ALT+ and one ALT-; for example, a DNA from previously characterized ALT+ tumors and ALT- tumors, either extracted from paraffin block or frozen sample;
- Products of the qPCR are quantified as well-known by the man skilled in the art, following the E_{Telo}^{- *CT(telo)*} / E_{36B4}^{- *CT(36B4)*} method, where the threshold cycle (*CT*) is the cycle at which the fluorescence level reaches a certain amount (the threshold) for each qPCR (CT(Telo) and CT36B4), and E stands for PCR efficiency and is determined for each gene according to the standard curves.

The telomere content value representative of the mean quantity of telomeric sequences in cells for each sample is obtained. This value is next normalized by internal controls obtained from DNA extracted from ALT+ and ALT- cell lines, as it is well known by the man skilled in the art. The resulting value is designated as "T-length" value.

Four threshold have been defined as a function of increasing TL : short, middle-long, long and very long. With these thresholds, the telomere length status of the glioma can be determined as follow:
- Long telomere, when the T-length value is superior to the threshold value "very long";
- Intermediate telomere, when the T-length value is comprised between the threshold values "middle-long" and "long"; and
- Short telomere, when the T-length value is inferior to the threshold value "short

In a specific embodiment of the invention, the *in vitro* process for classifying a glioama comprises the following steps:
a. Measuring, from a glioma sample, the following two parameters:
   i. The Alternative Lengthening of Telomeres (ALT) status ; and
   ii. The telomere length status;
b. Optionally, determining the isocitrate dehydrogenase genes mutation status (IDH status) of said glioma;
c. Optionally, determining the histological grade of said glioma;
d. Based on the data obtained in steps (a) (b) and (c), classifying said glioma in one of the five following classes: oligodendroglioma -like, glioblastoma IDHwt -like, glioblastoma IDHmt -like, low-grade astrocytoma -like, and other gliomas,
wherein the telomere length status in (a.ii) is measured by quantifying the telomeric DNA with a telomere-specific PCR, thereby obtaining a T-length value and selecting the telomere length status of the glioma.

In a specific embodiment of the invention, the ALT status and the T-length status are measured concomitantly with the same experimentation, comprising:
- the C-circle assay, and
- a telomere specific quantitative PCR.

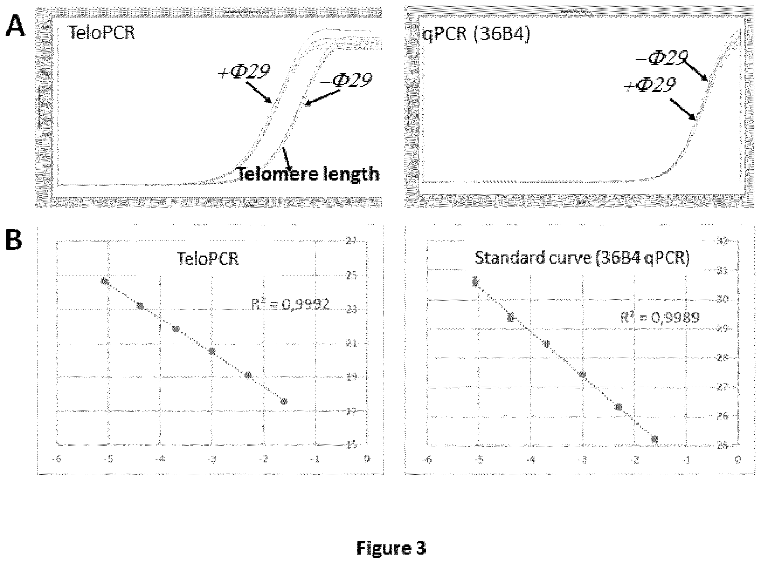

Figure 3 presents an example of standard curves that can be used for the calculation of the qPCR efficiency, and that are useful for determining both the C-circle value and the telomere length.

### IDH status

The parameter of IDH status has previously been described for the classification of gliomas.

In a specific embodiment of the invention, the IDH status in optional step (b) is determined by sequencing both genes encoding proteins IDH1 and IDH2.

Sequences of human isocitrate dehydrogenase proteins 1 and 2 are well known of the man skilled in the art, and can be found in proteins database such as GeneBank and OMIM.

Wild-type sequence of human IDH1 is represented in SEQ ID NO:1, and wild type sequence of human IDH2 is represented in SEQ ID NO: 2, as presented in table 1 below:

| | | |
|---|---|---|
| SEQ ID NO: 1 | | UniProtKB - O75874, OMIM 147700 |
| SEQ ID NO: 2 | | UniProtKB - P48735, OMIM 147650 |

The IDH status of the glioma cells is determined as follows:
- The glioma is said of "IDH mutated (IDHmt) status" in case of the identification of a point mutation of residue R132 in IDH1 protein, and/or of a point mutation of residue R172 in IDH2 protein, and
- The glioma is said of "IDH wild-type (IDHwt) status" in case of each of both proteins IDH1 and IDH2 present a wild-type sequence at the respective residues R132 and R172.

Step (b) of the process according to the invention is optional. Indeed, and as illustrated in figure 1, determination of the IDH status is particularly relevant in the following case: when the intermediate classification (after step (a)) is "GbmOD" i.e. an oligodendroglioma or a glioblastoma; indeed, a glioma presenting the following features:
∘ ALT status = ALT-; and
∘ T-length = intermediate ;
could be an oligodendroglioma or a glioblastoma.

In this case, a step (b) of determination of the IDH status is necessary for the final classification, where:
∘ If IDH status = IDHwt, then the glioma is a primary glioblastoma (GBM_IDHwt or GBMI);
∘ If IDH status = IDHmt, the glioma is an oligodendroglioma (OD), with a much better prognostic.

### Histological grading of the glioma

Optionally, the tumor grading is measured in step (c) on the basis of immuno-histochemical analyses of pictures of said tumor, thereby selecting the tumor grade of the glioma from stage II, III or IV.

These grades are defined in the actual WHO classification of gliomas, and are based on the presence of the following criteria of the tumor cells: mitosis, vascular proliferation and necrosis.

The grade of the glioma will advantageously be determined by a man skilled in the art such as a neuro-specialized physician.

When no solid glioma sample is available, for example in the case of the implementation of the process on glioma DNA extracted from blood or cerebrospinal fluid of the patient, the histological grading of the tumor is not determined.

### Reclassification of a glioma

As previously presented, the subclass of "astrocytoma IDHwt" as determined by WHO's classification is particularly heterogeneous and difficult to classify.

Often, these gliomas are not actually conventional "astrocytomas" as defined by the WHO classification.

Therefore, the median overall survival is highly variable with a median of 2.5 years, which is much more severe than for classical astrocytoma. Moreover, therapeutic options are not defined by guidelines and might be wrongly chosen.

Therefore, there is a need for reclassifying these specific gliomas with a novel combination of parameters representative of the nature of said glioma.

In a particular aspect, the invention concerns an *in vitro* process for reclassifying a glioma of the class "astrocytoma IDHwt" according to the WHO's classification, comprising the following steps:
a. Measuring, for said astrocytoma IDHwt, the following two parameters :
   i. The Alternative Lengthening of Telomeres (ALT) status ; and
   ii. The telomere length status;
b. Optionally, determining the histological grade of said glioma;
c. Based on the data obtained in steps (a) and (b), classifying said "astrocytoma IDHwt -like" in one of the following classes: oligodendroglioma-like, glioblastoma IDHwt-like, glioblastoma IDHmt-like, ow-grade astrocytoma-like, and other gliomas.

In this embodiment, the IDH status of the glioma is known before the implementation of the process and therefore is not assessed again.

In this process, the histological grade of the "astrocytoma IDHwt" is usually known before the implementation of the process. However, optionally, the histological grade of the "astrocytoma IDHwt" might be determined during the process in step (b).

Although the IDH status of this kind of glioma is IDHwt, the glioma might be re-classified in a class such as "glioblastoma_IDHmt-like" or "low-grade astrocytoma -like", although tumors in this class present an IDHmt status. Nevertheless, it is understood that in this case, the glioma previously classified as "IDHwt astrocytoma" keeps its status of IDHwt, but is categorized in this astrocytoma class because the patient affected with said glioma would present the same median overall survival than those affected with such astrocytoma.

In this implementation of the process, further steps could be included such as determination of the status of TERT (mutated or wild-type) (Vinagre et al., 2013) and determination of the status of ATRX (present or absent) (Nandakumar et al., 2017).

### Uses of the process of the invention in therapy

The present invention also relates to a process for choosing a therapeutic strategy for treating a glioma, comprising the steps of:
a) Implementing the *in vitro* process for classifying said glioma as presented above, and
b) Based on said classification, choosing the more adapted therapeutic strategy for the patient affected by said glioma.

These therapies can be the actual "gold standard" therapies: radiotherapy with a concomitant or adjuvant chemotherapy, with chemotherapeutic agents such as Temozolomide or PCV, a combination treatment for brain tumors including procarbazine, lomustine, and vincristine.

The present invention also relates to a process for adapting a therapeutic strategy for treating a glioma, comprising the steps of:
a) Implementing the *in vitro* process for classifying said glioma as presented above, and
b) Based on said classification, adapting the therapeutic strategy for the patient affected by said glioma.

A promising therapeutic approach for the treatment of gliomas is the administration of inhibitors of any telomere maintenance mechanism. In particular, it has been shown that telomerase inhibitors increase the response to radiotherapy in a murine orthotopic model of human gliobastoma (Ferrandon *et al.,* 2015).

Thus, the present invention relates to an inhibitor of at least one telomere maintenance mechanism (TMM) for its use in the treatment of a glioma, wherein said glioma has been previously classified according to the process of the invention.

Advantageously, the determination of the ALT status of the glioma allows the practician to choose between TMM inhibitors specific of the ALT mechanism, or specific of the telomerase reactivation mechanism.

### Implementation of the process with a computer

In a specific implementation of these processes, data obtained in steps (a) (b) and (c) are recorded on a computer device, into a software program that is configured to memorize said data and to execute steps to classify the glioma in function of said parameters data.

Accordingly, the present invention also concerns a computer program product comprising code instructions for implementing a process as described above, for classifying a glioma.

### Kit for the implementation of the processes

The present invention also concerns a kit for the implementation of the processes as described, comprising:
- Reagents suitable for performing a C-circle assay;
- Reagents suitable for performing a Telomere-specific PCR; and
- Adequate internal controls comprising genomic DNA from ALT+ cells and from ALT- cells.

In particular, internal controls are chosen among cell lines expressing the telomerase (ALT- cells), such as breast cancer-derived cells, and among cell lines using the process of "Alternative lengthening of telomere" (ALT+ cells), such as osteosarcoma cell lines.

Other useful internal controls can be also present in said kit for the implementation of the processes of the invention.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### Example 1. C-circle reaction

Rolling circle amplification of C-circle is performed as described in (Henson *et al.,* 2009) and (Henson *et al.,* 2014). Briefly, 3.2µl of total genomic DNA (5ng/µL) were incubated for 18h at 30°C with 3,75 units of ϕi29 DNA polymerase (New England Biolabs) (0.375µL of 10U/µL), in 0.2µg/µL of BSA, 0.1% Tween, 4µM DTT (Dithiothreitol), 1mM dNTP, 1µL of 10X NEB buffer. Enzyme is heat-inactivated at 65°C for 20 min. The same reaction is performed without the enzyme ϕ29 (ϕ-).

For each experiment, two internal controls are added, namely TA and ALT. TA and ALT correspond to total genomic DNA extracted from HeLa (ALT-) and U2OS (ALT+) cell lines respectively.

The 10µL of ϕ- and cp+ reactions are then diluted by adding 30µL of water (molecular biology grade), 5µL are used to performed each qPCR reaction.

### Example 2. qPCR experiment

Oligonucleotides for the qPCR reaction have been previously described in (Gil *et al*., 2004) and (Lau *et al*., 2013).

The sequence for oligonucleotides used in the qPCR are presented in table 2:

| | | |
|---|---|---|
| SEQ ID NO: 3 | 5'-CGGTTTGTTTGGGTTTGGGTTTGGGTTTGGGTTTGGGTT-3' | forward Tel1a |
| SEQ ID NO: 4 | 5'-GGCTTGCCTTACCCTTACCCTTACCCTTACCCTTACCCT-3' | reverse primer tel2B |
| SEQ ID NO: 5 | 5'-CAGCAAGTGGGAAGGTGTAATCC-3' | RPL0/36B 4 forward primer |
| SEQ ID NO: 6 | 5'-CCATTCTATCATCAACGGGTACAA-3' | RPL0/36B 4 reverse primer |

Telo-PCR and qPCR against 36B4 are run in duplicate for each condition ϕ- and ϕ+, on a 480 Light Cycler Thermocycler (Roche, Houwald, Luxembourg), in 1X final LightCycler®

FastStart DNA Master SYBR Green I (10|jL), 200nM final of TeloPCR-specific primers or 300 nMm final of 36B4-specific primers. Details of thermocycling conditions are detailed below

For each qPCR, the exact conditions are summarized in tables 3 and 4 below:

**Table 3. TeloPCR**

| Analysis Mode | Cycles | Segment | Target Temperature | Hold time | Acquisition Mode |
|---|---|---|---|---|---|
| Pre Incub ation | | | | | |
| None | 1 | | 95°C | 10 min | none |

| Amplification | | | | | |
|---|---|---|---|---|---|
| Quantification | 32 | Denaturation | 95°C | 5 s | none |
| | | Annealing | 56 °C Rate = 4°C/s | 10 s | none |
| | | Extension | 72°C | 60 s | single |

| Melting Curve | | | | | |
|---|---|---|---|---|---|
| Melting Curves | 1 | Denaturation | 95 °C | 0 s | none |
| | | Annealing | 65 °C | 15 s | none |
| | | Melting | 95 °C Ramp Rate = 0,1 °C/s | 0 s | continuous |

| Cooling | | | | | |
|---|---|---|---|---|---|
| none | 1 | | 40 °C | 30 s | none |

**Table 4. PCR36B4**

| Analysis Mode | Cycles | Segment | Target Température | Hold time | Acquisition Mode |
|---|---|---|---|---|---|
| Pre Incubation | | | | | |
| None | 1 | | 95°C | 10 min | none |

| Amplification | | | | | |
|---|---|---|---|---|---|
| Quantification | 36 | Denaturation | 95°C | 5s | none |
| | | Annealing | 58 °C | 10 s | none |
| | | Extension | 72°C | 40 s | single |

| Melting Curve | | | | | |
|---|---|---|---|---|---|
| Melting Curves | 1 | Denaturation | 95°C | 0 s | none |
| | | Annealing | 65 °C | 15 s | none |
| | | Melting | 95 °C Ramp Rate = 0,1 °C/s | 0 s | continuous |

| Cooling | | | | | |
|---|---|---|---|---|---|
| none | 1 | | 40 °C | 30 s | none |

### Example 3. Data analysis, normalization and classification

The fluorescence in logarithmic scale is analyzed as a function of PCR cycle, the threshold is set as half the high of the linear fraction of the amplification curves (all experiments). Intersection between the threshold of amplification curve gives the CT for each reaction.

Efficiency of TeloPCR (Eₜₑₗₒ) and 36B4 (E_{36B4}) PCR are respectively 1.70111 and 1.9672.

For each reaction, the following value is calculated : Eₜₑₗₒ^{-CT}/ E_{36B4}^{-CT}, and annotated as ϕ+ and ϕ- as a function of the initial circle reaction.

ϕ- correspond to the telomere length (T-Length)
Cᵣ correspond to the Circle score and is calculated as follow : ϕ+ / ϕ-

The difference of T-Length between the two internal controls Δ is calculated and correspond to x units (determined for each batch of controls) with Δ= ϕ-_{HeLa} - ϕ-_{U2OS}
TLₙₒᵣₘ is then calculated for each sample as follow : ϕ-/(Δ/x)
A standard curve for calculation of qPCR efficiency is presented in figure 3.

As shown in figure 1, the first step (a) of the algorithm allows the classification of the tumors into 4 main categories:
- OD (oligodendroglioma),
- GbmOD (glioblastoma (GBM_IDHwt in figure 1) or OD),
- A_GBM_IDHmt (AD (AII_IDHmt in figure 1), AA (AIII_IDHmt in figure 1), or GBMII (GBM_IDHmt in figure 1), and
- others.

GbmOD category is then subclassified in step (b) according to the IDH status:
- If the tumor is IDHwt : GBM_IDHwt or
- If the tumor is IDHmt: OD.

All glioma are then classified according to their grade in step (c). In particular, tumors of the categorie "A_GBM_IDHmt" will be subclassified, according to their grade, into AII-IDHmt like (AD), AIII_IDHmt like (AA) or GBM_IDHmt like (GBMII).

An example of equations is given below; however, the indicated numbers have been defined for this experiment only, and are not limitating the scope of the claimed invention.

### 1^{st} step (a)

A=(SI(TLₙₒᵣₘ>4;"A_GBMII";SI(ET(Cᵣ>1,5;TLₙₒᵣₘ>0,8);"A_GBMII";SI(ET(TLₙₒᵣₘ<0,66; Cᵣ<1,9);"OD";SI(ET(Cᵣ <1,28;ET(TLₙₒᵣₘ>0,66; TLₙₒᵣₘ<3));"GbmOD";SI(Cᵣ >2;"A_GBMII";"other"))))))

### 2^{nd} step (b) with IDH status (MT or WT)

B=SI(ET(A="GbmOD";IDH="MT");"OD";(SI(ET(A="GbmOD";IDH="WT");"GBM";A)))

### 3^{rd} step with grading G (II. III, IV)

C=SI(ET(B="A_GBMII";G="III");"AA";SI(ET(B="A_GBMII";G="II");"AD";SI(ET(B="A_GBMII ";G="IV");"GBMII";B)

### Example 4. Obtained results

First, only 180 patients with molecular biomarkers in agreement with the immuno-histochemical classification (i.e. with the exception of anaplastic astrocytoma IDHwt) were analyzed.

These gliomas have been classified according to the standard algorithm according to the WHO 2016 classification (upper line), or with the algorithm of the invention (bottom line) and are presented in figure 4A.

The algorithm of the invention allows a separation into five subclasses: "oligodendroglioma-like", "glioblastoma IDHwt-like", "glioblastoma IDHmt-like", "low-grade astrocytoma -like" and "other" (the code is depicted in the legend).

Both processes of classification are concordant for 93.5% of gliomas. However for 3.8% of gliomas, the classfications are different (noted misclassified). With the algorithm of the invention, 2.7% of gliomas are classified as "others".

In conclusion, with the classification process according to the invention:
∘ For 94% of the gliomas, the classification was the same with the standard algorithm than with the process of the invention;
∘ 3.8% of the gliomas were differently classified (noted "misclassified") - their re-classification was relevant with the OS and DFS curves presented in figure 5;
∘ 2.7% of the gliomas are classified in the category "others".

Figure 4B shows the obtained results when considering only the 29 gliomas with discordant molecular biomarkers and immuno-histochemical classification.

In this case, the classification obtained with the algorithm of the invention is in agreement with the standard process of classification for 72% of gliomas.

The figure 5 presents a comparison of the curves of median overall survival (OS) and median disease-free survival (DFS) obtained after standard classification and classification according to the process of the invention

The overall survival (OS, upper curves) and the disease free survival (DFS, bottom curves) have been followed for each patient.

210 patients were analyzed, classified as follow according to the standard classification: 74 low-grade astrocytoma, 24 glioblastoma IDHwt, 57 glioblastoma IDHmt, and 55 oligodendroglioma.

Among these patients, 29 have discordant molecular parameters as regard with the immuno-histological classification.

The algorithm of the invention allows a separation into five subclasses: oligodendroglioma-like, glioblastoma IDHwt-like, glioblastoma IDHmt-like, low-grade astrocytoma -like (A_IDHmt like), and other gliomas.

Note that the curves are highly similar regarding the overall survival, and that the algorithm is even more discriminant that the standard classification considering the low grade astrocytoma and the oligodendroglioma.

### REFERENCES

WO 2017/127803
Louis DN, Perry A, Reifenberger G, von Deimling A, Figarella-Branger D, Cavenee WK, Ohgaki H, Wiestler OD, Kleihues P, Ellison DW. The 2016 World Health Organization Classification of Tumors of the Central Nervous System: a summary. Acta Neuropathol. 2016 Jun;131(6):803-20. doi: 10.1007/s00401-016-1545-1. Epub 2016 May 9. Review.
Henson JD, Cao Y, Huschtscha LI, et al. DNA C-circles are specific and quantifiable markers of alternative-lengthening-of-telomeres activity. Nat. Biotechnol. 2009;27(12):1181-1185.
Henson JD, Lau LM, Koch S, et al. The C-Circle Assay for alternative-lengthening-of-telomeres activity. Methods San Diego Calif. 2017;114:74-84.
Gil ME, Coetzer TL. Real-time quantitative PCR of telomere length. Mol. Biotechnol. 2004;27(2): 169-172.
Inda MM, Fan X, Muñoz J, Perot C, Fauvet D, Danglot G, Palacio A, Madero P,Zazpe I, Portillo E, Tuñón T, Martínez-Peñuela JM, Alfaro J, Eiras J, Bernheim A,Castresana JS. Chromosomal abnormalities in human glioblastomas: gain in chromosome 7p correlating with loss in chromosome 10q. Mol Carcinog. 2003 Jan;36(1):6-14. PubMed PMID: 12503074.
Vinagre J, Almeida A, Pópulo H, Batista R, Lyra J, Pinto V, Coelho R, Celestino R, Prazeres H, Lima L, Melo M, da Rocha AG, Preto A, Castro P, Castro L, Pardal F, Lopes JM, Santos LL, Reis RM, Cameselle-Teijeiro J, Sobrinho-Simões M, Lima J, Máximo V, Soares P. Frequency of TERT promoter mutations in human cancers. Nat Commun. 2013;4:2185.
Nandakumar, P., Mansouri, A., & Das, S. The Role of ATRX in Glioma Biology. Frontiers in oncology, 7, 236 (2017).
Ferrandon S, Malleval C, El Hamdani B, et al. Telomerase inhibition improves tumor response to radiotherapy in a murine orthotopic model of human glioblastoma. Mol Cancer. 2015;
Lau LM, Dagg RA, Henson JD, Au AY, Royds JA, Reddel RR. Detection of alternative lengthening of telomeres by telomere quantitative PCR. Nucleic Acids Res. 2013 Jan;41 (2):e34.

## Claims

1. An *in vitro* process for classifying a glioma, comprising the following steps :
a. Measuring, from a glioma sample, the following two parameters:
i. The Alternative Lengthening of Telomeres (ALT) status ; and
ii. The telomere length status;
b. Optionally, determining the isocitrate dehydrogenase genes mutation status (IDH status) of said glioma;
c. Optionally, determining the histological grade of said glioma;
d. Based on the data obtained in steps (a) (b) and (c), classifying said glioma in one of the five following classes: oligodendroglioma -like, glioblastoma IDHwt -like, glioblastoma IDHmt -like, low-grade astrocytoma -like, and other gliomas.

2. The *in vitro* process according to claim 1, wherein each class of glioma is defined by a median time of overall survival of the patients affected by said glioma.

3. The *in vitro* process according to claim 1 or 2, wherein the steps (a) and (b) are realized on the basis of DNA extracted from a glioma sample that has been conserved in paraffin or frozen.

4. The *in vitro* process according to anyone of claims 1 to 3, wherein the steps (a) and (b) are realized on the basis of DNA extracted from a blood sample or from a cerebrospinal fluid sample of a patient having said glioma.

5. The *in vitro* process according to anyone of claims 1 to 4, wherein the value of each one of the parameters (a.i) and (a.ii) is compared to at least one threshold value.

6. The *in vitro* process according to anyone of claims 1 to 5, wherein the ALT status in (a.i) is measured by performing a C-circle assay coupled to a telomere-specific PCR, thereby obtaining a C-circle value and selecting the ALT status of the glioma from:
- ALT++, when the C-circle value is superior to the threshold value "high";
- ALT+, when the C-circle value is superior to the threshold "positive";
- ALT intermediate, when the C-circle value is inferior to threshold values "intermediate", and
- ALT-, when the C-circle value is inferior to the threshold value "negative".

7. The *in vitro* process according to anyone of claims 1 to 6, wherein the telomere length status in (a.ii) is measured by quantifying the telomeric DNA with a telomere-specific PCR, thereby obtaining a T-length value and selecting the telomere length status of the glioma from:
- Long telomere, when the T-length value is superior to the threshold value "very long";
- Intermediate telomere, when the T-length value is comprised between the threshold values "long" and "middle-long"; and
- Short telomere, when the T-length value is inferior to the threshold value "short".

8. The *in vitro* process according to anyone of claims 1 to 7, wherein the IDH status in step (b) is determined by sequencing both genes encoding proteins IDH1, whose wild-type sequence is represented in SEQ ID NO.1, and IDH2, whose wild-type sequence is represented in SEQ ID NO. 2, thereby selecting the IDH status of the glioma from:
- IDH mutated (IDHmt) status in case of a mutation of residue R132 in IDH1 protein, and/or a mutation of residue R172 in IDH2 protein, or
- IDH wild-type (IDHwt) status in case of both proteins IDH1 and IDH2 present a wild-type sequence at the respective residues R132 and R172.

9. The *in vitro* process according to anyone of claims 1 to 8, wherein the histological grade in step (c) is determined on the basis of immuno-histochemical analyses of said glioma sample, thereby selecting the histological grade of said glioma from stage II, III or IV.

10. An *in vitro* process for reclassifying a glioma of the class "astrocytoma" with a IDH status "IDHwt", comprising the following steps :
a. Measuring, for said astrocytoma IDHwt, the following two parameters:
i. The Alternative Lengthening of Telomeres (ALT) status ; and
ii. The telomere length status;
b. Optionally, determining the histological grade of said glioma;
c. Based on the data obtained in steps (a) and (b), classifying said "astrocytoma IDHwt-like" in one of the following classes: oligodendroglioma-like, glioblastoma IDHwt-like, glioblastoma IDHmt-like, low-grade astrocytoma -like and other gliomas.

11. A process for choosing a therapeutic strategy for treating a glioma, comprising the steps of :
a) Implementing the *in vitro* process for classifying said glioma according to anyone of claims 1 to 10; and
b) Based on said classification, choosing the more adapted therapeutic strategy for the patient affected by said glioma.

12. A process for adapting a therapeutic strategy for treating a glioma, comprising the steps of :
a) Implementing the *in vitro* process for classifying said glioma according to anyone of claims 1 to 10; and
b) Based on said classification, adapting the therapeutic strategy for the patient affected by said glioma.

13. A computer program product comprising code instructions for implementing a process according to anyone of claims 1 to 10, for classifying a glioma.

14. A kit for the implementation of the processes according to anyone of claims 1 to 12, comprising:
- Reagents suitable for performing a C-circle assay;
- Reagents suitable for performing a Telomere-specific PCR; and
- Adequate internal controls comprising genomic DNA from ALT+ cells and from ALT- cells.

15. Inhibitor of at least one telomere maintenance mechanism for its use in the treatment of a glioma, wherein said glioma has been previously classified according to the process according to anyone of claims 1 to 10.


SEQUENCE LISTING

<110>  HOSPICES CIVILS LYON
       UNIVERSITE CLAUDE BERNARD LYON 1

<120>  PROCESS FOR CLASSIFICATION OF GLIOMA

<130>  B74923EP D38991

<160>  6

<170>  PatentIn version 3.5

<210>  1
<211>  414
<212>  PRT
<213>  Homo Sapiens

<400>  1

<210>  2
<211>  452

<212>  PRT
<213>  Homo Sapiens

<400>  2

<210>  3
<211>  39
<212>  DNA
<213>  Artificial

<220>
<223>  Oligonucleotide

<400>  3
cggtttgttt gggtttgggt ttgggtttgg gtttgggtt                              39


<210>  4
<211>  39
<212>  DNA
<213>  Artificial

<220>
<223>  Oligonucleotide

<400>  4
ggcttgcctt acccttaccc ttacccttac ccttaccct                              39


<210>  5
<211>  23
<212>  DNA
<213>  Artificial

<220>
<223>  Oligonucleotide

<400>  5
cagcaagtgg gaaggtgtaa tcc                                               23


<210>  6
<211>  24
<212>  DNA
<213>  Artificial

<220>
<223>  Oligonucleotide

<400>  6
ccattctatc atcaacgggt acaa                                              24